(19) Europäisches Patentamt
European Patent Office
Office européen des brevets

(11) Publication number: **0 350 471 A2**

(12) **EUROPEAN PATENT APPLICATION**

(21) Application number: **89850207.5**

(22) Date of filing: **19.06.89**

(51) Int. Cl.[5]: **C 07 C 409/40**
C 07 C 409/44,
C 07 C 407/00, C 11 D 3/39,
C 07 C 309/13

(30) Priority: **05.07.88 SE 8802506**

(43) Date of publication of application:
**10.01.90 Bulletin 90/02**

(84) Designated Contracting States:
**AT BE CH DE ES FR GB GR IT LI LU NL SE**

(71) Applicant: **BEROL NOBEL NACKA AB**
**Box 11536**
**S-100 61 Stockholm (SE)**

(72) Inventor: **Palicka, Jadwiga**
**Ekbackevägen 26**
**S-181 46 Lidingö (SE)**

**Legnerfält, Björn Allan**
**Kungshamra 15 A**
**S-171 70 Solna (SE)**

**Walding, Jan**
**Färgargardstorget 68**
**S-116 43 Stockholm (SE)**

(74) Representative: **Schöld, Zaid**
**Nobel Industries Sweden AB Patent Department Box 11554**
**S-100 61 Stockholm (SE)**

# (54) New compounds, their preparation and use.

(57) New compounds having the general formula

$$R-(A)_n-[\underset{B}{N}-(CHR_1)_x]_y-\underset{B}{N}-Q$$

wherein R is a higher hydrocarbon group, Q is the group $-R_2-S(O)_2-O-M$, $-R_2-S(O)_2-O-O-M$ or the group $-R_2-C(O)-O-O-M$ and B is hydrogen or has the same meaning as Q. The compounds are useful for enhancing the bleaching performance at washing and give compounds having surface activity as decomposition products.

EP 0 350 471 A2

## Description

### New compounds, their preparation and use

The present invention relates to new compounds and the use of the compounds for enhancing the bleaching effect at washing. More particularly the invention relates to compounds which can be characterized as sulfonated, persulfonated or percarboxylated amphoteric compounds. The invention also relates to a method for the preparation of the compounds and to the use of the compounds.

Amphoteric compounds which can be characterized by the general formula

$$R-(A)_n-[\underset{B}{N}-(CHR_1)_x]_y-\underset{B}{N}-D$$

wherein R is a higher hydrocarbon group, A is the group (C(O)) or ($OCH_2CH_2$) and n is 0 or 1, $R_1$ is hydrogen or a lower alkyl group, x is 2 or 3, y is 0 to 4, D is a group $-R_2COOM$ wherein $R_2$ is an alkylene group and M is hydrogen or another cation and B is hydrogen or a group D are per se known. Compounds of this type are disclosed in the European patent applications 160507, 162600 and 214868. The compounds are used for surface activity and for their antimicrobial properties. Amphoteric compounds of the above type wherein D is the group $-CH_2SO_3M$ are also known from the British patent 1401984, and used for improving textiles, paper and leather.

The compounds of the present invention can be considered as derivatives of the above mentioned amphoteric compounds wherein the group D is a group Q of formula

$$-R_2-\overset{O}{\underset{O}{\overset{\|}{\underset{\|}{S}}}}-OM, \qquad -R_2-\overset{O}{\underset{O}{\overset{\|}{\underset{\|}{S}}}}-O-O-M \quad \text{or}$$

$$-R_2-\overset{O}{\overset{\|}{C}}-O-O-M.$$

The per-compounds of the present invention have bleaching effect and can be used as bleaching agents at washing. The compounds which contain one or more sulfonic acid groups can be used as bleach activators, ie for bleaching in combination with a hydrogen peroxide source. The compounds of the present invention are particularly advantageous in that they as decomposition products at bleaching give a surface active compound having both detergency effect and antimicrobial properties.

The present invention thus relates to new compounds which can be characterized by the general formula

$$R-(A)_n-[\underset{B}{N}-(CHR_1)_x]_y-\underset{B}{N}-Q$$

wherein R is a hydrocarbon group having 7 to 22 carbon atoms, A is the group (C(O)) or the group ($OCH_2CH_2$) and n is 0 or 1, R is hydrogen or a lower alkyl group, x is 2 or 3, y is an integer of 1 to 4, Q is the group $-R_2-S(O)2-O-M$, $-R_2-S(O)2-O-O-M$ or the group -R2-C(O)-O-O-M wherein $R_2$ is an alkylene group with 2 to 6 carbon atoms, which can contain a hydroxy group, and M is hydrogen or an ion from the groups alkali metals, alkaline earth metals, ammonium or substituted ammonium or a group $-C(O)R_3$, wherein $R_3$ is an alkyl group and B is hydrogen or a group Q as defined.

In the compounds according to the invention R is a hydrocarbon group having 7 to 22 carbon atoms and suitably 12 to 20 carbon atoms. The hydrocarbon group R can be straight or branched, saturated or unsaturated and can also be a cycloalkyl-alkyl group, an arylalkyl or arylalkenyl group wherein the alkyl or alkenyl group contains at least 6 carbon atoms. It is preferred that R is an alkyl or alkenyl group. Compounds wherein A is a carbonyl group and compounds wherein n is 0 are preferred to those wherein A is the group ($OCH_2CH_2$). $R_1$ is hydrogen or a lower alkyl group, suitably having 1 to 6 carbon atoms and is preferably hydrogen or a methyl group, x is 2 or 3 and y is suitably 1, 2 or 3. The group $R_2$ is suitably an ethylene, propylene, 2-hydroxypropylene or butylene group. M is hydrogen, an ion as defined above and hereby substituted ammonium should be understood as for example mono-, di- or trihydroxyalkylammonium, such as mono-, di- or trihydroxyethylammonium, or the group $-C(O)R_3$. M is preferably hydrogen, sodium or magnesium or the group $-C(O)R_3$ wherein $R_3$ can have from 1 to 18 carbon atoms, suitably from 1 to 10. $R_3$ preferably has from 1 to 5 carbon atoms and is most preferably a methyl group. The compounds according to the invention are characteristic in that they contain a longer hydrocarbon group, at least two nitrogen groups and at least a group Q as defined above. The fact that the group $R_2$ contains 2 or more carbon atoms simplifies the peroxidation, at the preparation of the persulfonic acid compounds and percarbonic acid compounds respectively the in situ formation of persulfonic acid compounds from the sulfonic acid compounds in the presence of a hydrogen peroxide source. It is preferred that all the groups B in the compounds are groups Q as defined. It is of course also encompassed by the invention that the compounds can contain different groups Q. However, when the compounds contain more than one group Q it is preferred that they are the same groups Q or that they are combinations of the groups $-R_2-S(O)_2-O-M$ and -R2-S(O)2-O-O-M.

The most preferred compounds are those wherein n is 0, $R_1$ is hydrogen, x is 3, y is 1 or 2, preferably 1, and

all groups B are the same groups Q and $R_2$ in the groups Q are then preferably ethylene, propylene or butylene groups and M is preferably a sodium ion or the group -C(O)$R_3$ wherein $R_3$ is a lower alkyl group, preferably a methyl group. The hydrocarbon group R in these compounds is most preferably a C12 to C18 alkyl or alkenyl group, or mixtures of these.

Compounds according to the invention wherein one or more groups Q is -$R_2$-C(O)-O-O-M can be prepared starting from the corresponding amphoteric compound wherein one or more groups D is the group -$R_2$-C(O)-OH by reaction with a peroxidating compound such as for example $H_2O_2$, peracetic acid or perbenzoic acid. Hydrogen peroxide is suitably used and it is hereby advantageous to use a large molar excess of the hydrogen peroxide, up to about 100 to 1000 per cent. The actual reaction is carried out as per se previously known for this type of reaction at temperatures of from about 0 to about 80° C. As catalyst Lewis acids can be used, for example HCl, $AlCl_3$, $H_2SO_4$ and methanesulfonic acid and they can also be used as reaction medium. Otherwise water can be used as reaction medium. The reaction is normally finished in an hour or a couple of hours. The product is obtained as a clear liquid of low viscosity. Salts of the acid can be prepared using known methods for salt formation and compounds containing the group -C(O)$R_3$ can be prepared in a known manner by reaction with the appropriate anhydride, eg acetic acid anhydride.

Compounds wherein Q stands for the group -$R_2$-S(O)$_2$-O-M can suitably be prepared starting from a mono-, di- or polyamine of the general formula R - $(A)_n$ - [NH - $(CHR_1)_x]_y$ - $NH_2$, wherein R, A, $R_1$, n, x and y have the definitions given above. The amines can be reacted with for example dichloroethane or dibromoethane and the obtained reaction product subsequently reacted with a sulfite reagent. Compounds wherein $R_2$ is an alkylene group having 3 or more carbon atoms are preferred, and especially compounds wherein $R_2$ is a propylene or butylene group, and these can be prepared by reaction between the amine and the corresponding alkylsultone. The reaction is here suitably carried out in water and at a temperature of from about 60 to about 90° C for times up to about 24 hours. It is suitable to use a molar excess of the sultone with regard to the amine, up to about 10 per cent. As described earlier, the compounds can subsequently be converted to salts or to compounds containing the group -C(O)$R_3$.

Starting from the obtained product with groups Q = -$R_2$-S(O)$_2$-OH according to what has been disclosed above this can be further reacted with hydrogen peroxide or another peroxide donating compound, as above, to give compounds with groups -$R_2$-S(O)2-O-O-H which can then be converted into salts or into compounds containing the group -C(O)$R_3$. The reaction conditions at peroxidation correspond to those given for preparation of compound with groups -$R_2$-C(O)$_2$-O-H but as reaction medium here it is suitable to use an aprotic solvent, for example tetrahydrofuran or dimethylformamide.

As has been mentioned above compounds containing different groups Q can be prepared. When these are groups -$R_2$-S(O)$_2$-O-M and groups -$R_2$-S(O)$_2$-O-O-M the ratio is controlled by the degree of peroxidation while compounds containing both groups -$R_2$-C(O)-O-O-M and groups based on sulfonic acid or persulfonic acid can be prepared starting from the amine, as defined earlier, which is partly converted to the corresponding amphoteric compound by reaction with for example acrylic acid or chloroacetic acid whereafter the obtained product is reacted with sultone and then peroxidized.

The present invention also relates to the use of the new compounds described above for enhancing the bleaching performance at washing. Compounds according to the invention which contain groups

$$-R_2-\overset{O}{\overset{\|}{C}}-O-O-M \quad \text{or} \quad -R_2-\underset{\underset{O}{\|}}{\overset{\overset{O}{\|}}{S}}-O-O-M$$

have bleaching effect themselves while compounds containing groups

$$-R_2-\underset{\underset{O}{\|}}{\overset{\overset{O}{\|}}{S}}-O-M$$

are so-called bleach activators, or precursors, and give in the presence of a hydrogen peroxide source the bleaching persulfonic acid compound in the wash liquor. The compounds of the invention are very advantageous in this use since they both give bleaching effect at low temperatures compared with bleaching agents such as perborates and give decomposition products which are surface active compounds and contribute to the detergency performance. The bleaching compounds and the bleach activator compounds according to the invention give good bleaching effect already at temperatures of about 40° C and very good bleaching effect at temperatures of from about 60 to about 90° C. The bleach activator compounds are used in combination with per se known hydrogen peroxide sources and hereby sodium perborate monohydrate and sodium perborate tetrahydrate are preferably used but other hydrogen peroxide sources such as sodium percarbonate can also be used. The weight ratio of bleach activator to hydrogen peroxide source should suitably be within the range of from 1:2 to 4:1, preferably within the range of from 1:1 to 3:1 and most preferably within the range for from 2:1 to 3:1. As the bleach activator compounds also give a surface active effect comparatively high amounts of them can be used. The bleaching compounds can also, if desired, be

used in combination with the hydrogen peroxide sources, as disclosed above, which are conventionally used bleaching agents for bleaching effect at higher temperatures. The term washing is herein primarily used for washing of textiles, ie laundering, but other industrial or household applications where the bleaching effect at washing according to the invention can be useful are of course also conceivable.

The bleaching compounds or the bleach activator compounds of the invention can be used in per se known detergent compositions for laundering. In these compositions can be included surface active compounds such as nonionic, anionic, amphoteric and cationic tensides, builders such as polyphosphates, zeolites and NTA, optical brighteners, foam regulators, perfumes and colouring agents etc. Other bleach activators and/or bleaching agents can of course also be included. As the compounds of the invention at the bleaching give decomposition products which are surface active compounds not only a bleaching effect but also a surfactant effect is obtained at laundering.

The compounds of the invention can be incorporated in liquid or powder form detergent compositions, preferably in powder form compositions, which are produced in conventional manner and the bleaching compounds and the bleach activator compounds respectively are suitably included in amounts of from 5 to 40 per cent by weight, and preferably in amounts of from 15 to 30 per cent by weight, based on dry detergent composition.

The invention is further illustrated in the following examples which, however, are not intended to limit the same. Parts and per cent relate to parts by weight and per cent by weight unless otherwise stated.

Example 1

A compound of the formula R-NQ-$(CH_2)_3$-$NQ_2$ wherein Q is -$CH_2CH_2C(O)OOH$ and R is an alkyl residue from coco fatty acid, ie with a hydrocarbon chain length distribution of essentially C12/C14, was prepared according to the following: 5.0 g (11.0 mmoles) of the corresponding amphoteric compound with D = -$CH_2CH_2C(O)OH$ and 10.6 g (110 mmoles) of methanesulfonic acid were charged to a 100 ml three-neck flask equipped with reflux condenser, thermometer and a dropping funnel. When the amphoteric compound had been dissolved and the temperature by means of an ice bath had been adjusted to +10°C 3.2 g (66.2 mmoles) of 70% hydrogen peroxide were added drop by drop. The temperature was kept below +20°C all the time. When the hydrogen peroxide had been added the mixture was allowed to react for 1 hour. The obtained product was analyzed by means of thin layer chromatography adapted for identification of peracids. As the eluant ethanol/ammonia in a ratio of 9:1 was used and as reagent N,N-dimethyl-phenylene-diammonium dichloride was used. The substance immediately gave a red-rosy colour which is an indicator for peroxy acids.

Example 2

A compound R-NQ-$(CH_2)_3$-NQ-$(CH_2)_3$-NQ-$(CH_2)_3$-$NQ_2$ wherein Q is -$CH_2CH_2C(O)OOH$ and R an alkyl residue from tallow fatty acid, ie with a hydrocarbon chain length distribution of essentially C16/C18 was prepared principally according to the procedure disclosed in Example 1. 2.5 g (3,45 mmoles) of the corresponding amphoteric compound and 3.4 g (34.5 mmoles) of sulfuric acid were charged to the flask. When the amphoteric compound had been dissolved and the temperature adjusted to +10°C 1.7 g (34.5 mmoles) of 70% hydrogen peroxide were slowly added drop by drop. The temperature was kept below +20°C all the time. When all hydrogen peroxide had been added the solution was allowed to react for 1 hour. The product was analyzed by means of thin layer chromatography as in Example 1.

Example 3

A compound with the formula R-NQ-$(CH_2)_3$-$NQ_2$ wherein Q is the group -$(CH_2)_4S(O)_2OH$ and wherein R is an alkyl residue from coco fatty acid, ie with a hydrocarbon chain length distribution of essentially C12/C14, was prepared according to the following: The corresponding diamine was reacted with butanesultone. 20.0 g (84.5 mmoles) of the diamine, 34.5 g (253.5 mmoles) of 1,4-butanesultone and 80 ml of water were charged to a 250 ml two-neck flask equipped with reflux condenser and a thermometer. The reaction was allowed to proceed for 21 hours under nitrogen atmosphere. During the process the from the beginning turbid emulsion became clear. The product was recrystallized three times by dissolving it in decanol at +70 to +90°C and was then, at a lower temperature, precipitated with petroleum ether. 27.6 g of the product was obtained and this corresponds to a yield of 51%. The product was analyzed by IR and gave two characteristic peaks for -$S(O)_2$- at 1195-1170 $cm^{-1}$ and 1040 $cm^{-1}$ respectively and a characteristic peak for -S-O- at 605 $cm^{-1}$, which is entirely in accordance with data given in literature.

Example 4

In these tests the bleaching effect of the above prepared compounds was investigated. A solution was prepared containing 0.5 g of tannin, 5 g of sodium bicarbonate and 1.68 g of sodium perborate tetrahydrate ($NaBO_2.H_2O_2.3H_2O$) per liter. The pH of the solution was adjusted to about 10.5 with sodium hydroxide. For each test 100 ml of this solution, below called bleach solution, was used. The bleaching performance was evaluated by following the decolouring of the solution by means of UV-spectrophotometry. The tests were carried out at a temperature of +60°C and the wave length was 450 nm. The following solutions were investigated:

1) 100 ml of bleach solution + 451 mg (corresponding to 2.8 mg of active oxygen) of the compound according to Example 1.

2) 100 ml of bleach solution + 448 mg (corresponding to 2.8 mg of active oxygen) of the compound according to Example 2.

3) 100 ml of bleach solution + 113 mg of the compound according to Example 3 + 50 mg of sodium perborate monohydrate.

4) Comparison, 100 ml of bleach solution + 50 mg of sodium perborate monohydrate.

5) Comparison, 100 ml of bleach solution + 50 mg of sodium perborate monohydrate + 20 mg of the commercial bleach activator TAED.

The results are shown below as UV-absorbency after the indicated time and the differences between the values at the start and the end.

| Solution | UV-absorbency after given number of minutes | | | | | |
|---|---|---|---|---|---|---|
| | 0 | 10 | 20 | 30 | 40 | δ |
| 1 | 1.335 | 0.985 | 0.891 | 0.840 | 0.804 | 0.444 |
| 2 | 1.302 | 0.697 | 0.551 | 0.492 | 0.467 | 0.751 |
| 3 | 1.274 | 0.969 | 0.822 | 0.771 | 0.743 | 0.531 |
| 4 ref. | 1.314 | 1.110 | 1.010 | 0.964 | 0.916 | 0.398 |
| 5 ref. | 1.336 | 0.999 | 0.854 | 0.772 | 0.721 | 0.615 |

**Claims**

1. Compounds, characterized by the general formula$R - (A)_n - [\underset{B}{N} - (CHR_1)_x]_y - \underset{B}{N} - Q$

wherein R is a hydrocarbon group having 7 to 22 carbon atoms, A is the group (C(O)) or the group ($OCH_2CH_2$) and n is 0 or 1, $R_1$ is hydrogen or a lower alkyl group, x is 2 or 3, y is an integer of 1 to 4, Q is the group $-R_2-S(O)_2-O-M$, $-R_2-S(O)_2-O-O-M$ or the group $-R_2-C(O)-O-O-M$ wherein $R_2$ is an alkylene group with 2 to 6 carbon atoms, which can contain a hydroxy group, and M is hydrogen or an ion from the groups alkali metals, alkaline earth metals, ammonium or substituted ammonium or a group $-C(O)R_3$, wherein $R_3$ is an alkyl group and B is hydrogen or a group Q as defined.

2. Compounds according to claim 1, characterized in that n is 0 or A is the group (C(O)), R is an alkyl or alkenyl group with 7 to 22 carbon atoms, $R_1$ is hydrogen or a methyl group and y is 1, 2 or 3.

3. Compounds according to claim 1 or 2, characterized in that $R_2$ is an ethylene, propylene, hydroxypropylene or butylene group.

4. Compounds according to any of the preceding claims, characterized in that M is hydrogen, sodium, magnesium or the group $-C(O)R_3$ where $R_3$ is an alkyl group having 1 to 5 carbon atoms.

5. Compounds according to any of the preceding claims, characterized in that all groups B are groups $-R_2-S(O)_2-O-M$, $-R_2-S(O)_2-O-O-M$ and/or $-R_2-C(O)-O-O-M$.

6. A method for the preparation of compounds having the general formula

$R - (A)_n - [\underset{B}{N} - (CHR_1)_x]_y - \underset{B}{N} - Q$

wherein R is a hydrocarbon group having 7 to 22 carbon atoms, A is the group (C(O)) or the group ($OCH_2CH_2$) and n is 0 or 1, $R_1$ is hydrogen or a lower alkyl group, x is 2 or 3, y is an integer of 1 to 4, Q is the group $-R_2-C(O)-O-O-M$ wherein $R_2$ is an alkylene group with 2 to 6 carbon atoms, which can contain a hydroxy group, and M is hydrogen or an ion from the groups alkali metals, alkaline earth metals, ammonium or substituted ammonium or a group $-C(O)R_3$, wherein $R_3$ is an alkyl group and B is hydrogen or a group Q as defined, characterized in that an amphoteric compound of the general formula

$R - (A)_n - [\underset{B}{N} - (CHR_1)_x]_y - \underset{B}{N} - D$

wherein R, $R_1$, n, x and y have the above given meanings and D is a group $-R_2COOH$ and B is hydrogen or a group D as defined, wherein $R_2$ has the above given meaning, is reacted with a peroxidating compound for peroxidation whereafter the obtained product can be converted to a salt or to compounds containing groups $-R_2-C(O)-O-O-M$ where M is the group $-C(O)R_3$ by reaction with the corresponding acid anhydride.

7. A method for the preparation of compounds of the general formula

$R - (A)_n - [\underset{B}{N} - (CHR_1)_x]_y - \underset{B}{N} - Q$

wherein R is a hydrocarbon group having 7 to 22 carbon atoms, A is the group (C(O)) or the group ($OCH_2CH_2$) and n is 0 or 1, $R_1$ is hydrogen or a lower alkyl group, x is 2 or 3, y is an integer of 1 to 4, Q is the group $-R_2-S(O)_2-O-M$ and/or the group $-R_2-S(O)_2-O-O-M$ wherein $R_2$ is an alkylene group with 2 to 6 carbon atoms and M is hydrogen or an ion from the groups alkali metals, alkaline earth metals, ammonium or substituted ammonium or a group $-C(O)R_3$, wherein $R_3$ is an alkyl group and B is hydrogen or a group

Q as defined, characterized in that an amine with the general formula $R-(A)_n-[NH-(CHR_1)_x]_y-NH_2$ wherein R, $R_1$, A, n, x and y have the above given meanings is reacted with 1) a dihaloethane and the obtained reaction product is subsequently reacted with a sulfite reagent or 2) with an alkylsultone containing 3 to 6 carbon atoms whereafter the obtained compounds can be converted to salts or to compounds containing groups $-C(O)R_3$ by reaction with corresponding acid anhydride, or the obtained compounds are reacted with a peroxidating compound for peroxidation whereafter the obtained compounds in a corresponding manner can be converted to salts or compounds containing groups $-C(O)R_3$.

8. Use of compounds having the general formula

$$R - (A)_n - [\underset{\displaystyle B}{\overset{}{N}} - (CHR_1)_x]_y - \underset{\displaystyle B}{N} - Q$$

wherein R is a hydrocarbon group having 7 to 22 carbon atoms, A is the group (C(O)) or the group ($OCH_2CH_2$) and n is 0 or 1, $R_1$ is hydrogen or a lower alkyl group, x is 2 or 3, y is an integer of 1 to 4, Q is the group $-R_2-S(O)_2-O-M$, $-R_2-S(O)_2-O-O-M$ or the group $-R_2-C(O)-O-O-M$ wherein $R_2$ is an alkylene group with 2 to 6 carbon atoms, which can contain hydroxy groups, and M is hydrogen or an ion from the groups alkali metals, alkaline earth metals, ammonium or substituted ammonium or a group $-C(O)R_3$, wherein $R_3$ is an alkyl group and B is hydrogen or a group Q as defined, for enhancing the bleaching performance at washing.

9. Use of compounds according to claim 8 wherein Q is a group $-R_2-S(O)_2-O-M$ in combination with a hydrogen peroxide source for enhancing the bleaching performance at washing.